# FASCICULE DE BREVET EUROPEEN

(11) **EP 0 685 228 B1**
(45) Date de publication et mention de la délivrance du brevet: **30.06.1999**
(21) Numéro de dépôt: 95401080.7
(22) Date de dépôt: 10.05.1995
(51) Int. Cl.: A61K 7/42

(54) **Compositions cosmétiques antisolaires à base d'un mélange synergétique de filtres et utilisations**
Kosmetische Sonnenschutzmittel enthaltend eine synergistische Mischung von Filtern und Verwendungen
Cosmetic sunscreening compositions containing a synergic mixture of filters and uses

(30) Priorité: 03.06.1994 FR 9406829
(43) Date de publication de la demande: 06.12.1995
(73) Titulaire: L'OREAL, 75008 Paris (FR)
(72) Inventeur: Hansenne, Isabelle, F-75017 Paris (FR)
(74) Mandataire: Andral, Christophe André Louis

(56) Documents cités:
- EP-A- 0 518 772
- WO-A-91/11989

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins deux filtres particuliers, à savoir d'une part l'acide benzène 1,4-di(3-méthylidène-10-camphosulfonique) et, d'autre part, l'α-cyano-β, β-diphénylacrylate de 2-éthylhéxyle, ces deux filtres étant présents dans des proportions déterminées et convenant à l'obtention d'un effet de synergie au niveau des indices de protection conférés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, que la combinaison, dans des proportions comprises dans des limites bien déterminées, de deux filtres solaires particuliers et déja connus en soi dans l'état de l'art, permettait, du fait d'un effet de synergie remarquable, d'obtenir des compositions antisolaires présentant des indices de protection nettement améliorés, et en tous cas largement supérieurs à ceux qui peuvent être obtenus soit avec l'un ou l'autre des filtres utilisé seul, soit encore avec des associations contenant simultanément les deux filtres mais dans des rapports sortant du domaine de l'invention.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) de l'acide benzène 1,4-di(3-méthylidène-10-camphosulfonique), éventuellement sous forme partiellement ou totalement neutralisée, à titre de premier filtre, et (ii) de l'α-cyano-β, β-diphénylacrylate de 2-éthylhéxyle à titre de second filtre, lesdits premier et second filtres étant présents dans lesdites compositions dans une proportion produisant une activité synergique.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaitront à la lecture de la description détaillée qui va suivre.

L' acide benzène 1,4-di(3-méthylidène-10-camphosulfonique) et ses différents sels (composé A), décrits notamment dans les demandes de brevets FR-A- 2 528 420 et FR-A- 2 639 347, sont des filtres déja connus en soi (filtres dits à large bande) capables en effet d'absorber les rayons ultraviolets de longueur d'ondes comprises entre 280 et 400 nm, avec des maxima d'absorption compris entre 320 et 400 nm, en particulier aux alentours de 345 nm. Ces filtres répondent à la formule générale (I) suivante : 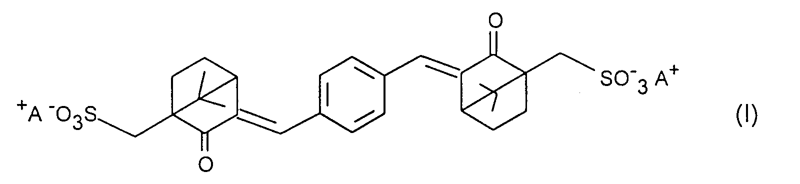 dans laquelle A désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺. Il est bien entendu que les composés de formule (I) ci-dessus peuvent donner lieu à l'isomère "cis-trans" autour d'une ou plusieurs double(s) liaison(s) et que tous les isomères rentrent dans le cadre de la présente invention.

De même, l'α-cyano-β, β-diphénylacrylate de 2-éthylhéxyle (composé B), encore appelé octocrylène, est un filtre lipophile liquide déja connu en soi pour son activité dans l'UV-B. Il s'agit d'un produit qui est disponible commercialement, et est vendu notamment sous la dénomination de "UVINUL N 539" par la Société BASF. Il répond à la formule (II) suivante : 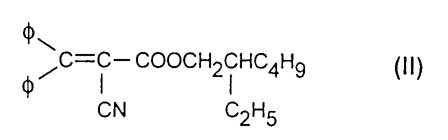 dans laquelle φ désigne un radical phényle.

Le composé A peut être présent dans les compositions selon l'invention à une concentration comprise entre 0,2 et 10 % en poids par rapport au poids total de la composition et le composé B peut être, quant à lui, présent à des teneurs comprises entre 0,5 et 20 % en poids, toujours par rapport au poids total de la composition ; de préférence, la teneur globale du mélange entre le composé A et le composé B n'excède pas 25 % du poids total de la composition finale. Comme indiqué précédemment, selon une caractéristique essentielle de la présente invention, il convient que ces deux composés soient tous deux présents dans la composition finale dans une proportion respective telle qu'un effet de synergie au niveau de l'indice de protection conféré par l'association résultante, soit obtenu de manière notable, substantielle et significative.

Ainsi, et plus précisément, il a été constaté dans la pratique que l'effet de synergie susmentionné entre le composé A et le composé B apparait généralement de façon remarquable dans un rapport pondéral [(composé B) / (composé A)] qui est compris entre 0,25 et 8 environ. De préférence, selon la présente invention, ce rapport est compris entre 0,5 et 7, et encore plus préférentiellement entre 1 et 5. On notera que la plage exacte des rapports pondéraux dans laquelle un effet de synergie optimal est effectivement atteint peut varier légèrement selon la nature du composé A mis en oeuvre (forme acide ou au contraire plus ou moins salifiée et type de sels).

En outre, et d'une manière générale, on notera que les concentrations et rapports en composés A et B sont choisis de manière telle que l'indice de protection solaire de la composition finale soit de préférence d'au moins 2.

Enfin, toujours selon un mode préféré de réalisation de la présente invention, le support cosmétiquement acceptable dans lequel se trouvent contenus les différents composés A et B, est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir ce ou ces éventuels composés complémentaires, en particulier les filtres solaires complémentaires, et/ou leurs quantités de manière telle que l'effet de synergie (au niveau des indices de protection conférés) qui est attaché à l'association filtrante binaire conforme à l'invention ne soit pas, ou substantiellement pas, altéré par la ou les adjonctions envisagées.

A cet égard, on notera qu'il a été décrit dans la demande de brevet EP-A-518 772 (exemple 3) une émulsion antisolaire comprenant, à titre de filtres, une association ternaire constituée de 2% d'acide benzène 1,4-di(3-méthylidène-10-camphosulfonique), 6% d'α-cyano-β, β-diphénylacrylate de 2-éthylhéxyle (〈〈 UVINUL N539 〉〉) et 2% de 4-tert.butyl-4'méthoxy-dibenzoylméthane (〈〈 PARSOL 1789 〉〉). Toutefois, commme le montreront les exemples ci-après, la présence dans la composition décrite du PARSOL 1789 inhibe totalement l'effet de synergie qui serait normalement attaché à l'association filtrante binaire restante si elle était mise en oeuvre seule.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1

On a préparé diverses formulations antisolaires se présentant sous la forme d'une émulsion de type huile-dans-eau et contenant (les quantités sont exprimées en % poids par rapport au poids total de la composition) :

| | |
|---|---|
| a) un premier filtre A qui était l'acide benzène 1,4-di(3-méthylidène-10-camphosulfonique), à raison de | x % |
| b) un deuxième filtre B qui était l'α-cyano-β, β-diphénylacrylate de 2-éthylhéxyle ("UVINUL N 539"), à raison de y | % |
| c) Mélange d'alcool cétylstéarylique et d'alcool cétylstéarylique oxyéthyléné à 33 moles d'oxyde d'éthylène vendu sous la dénomination "Sinnowax AO" par HENKEL (émulsionnant) | 7 % |
| d) Mélange de mono- di et tristéarate de glycérol (co-émulsionnant) | 2 % |
| e) Triglycérides d'acides gras en C8-C12 ("MIGLYOL 812") | 2 % |
| f) Polydiméthylsiloxane | 1,5 % |
| g) Alcool cétylique | 1,5 % |
| h) Eau | qsp 100 % |

ces formulations (numérotées 1 et 2 pour celles conformes à l'invention) présentant des rapports pondéraux y/x entre les filtres B et A différents ; à chacune de ces formulations, on a fait en outre correspondre des formulations comparatives qui : soit ne contenaient que le filtre A à la concentration pondérale x + y , soit ne contenaient que le filtre B également à la concentration pondérale de x + y , soit contenaient les filtres A et B toujours à la concentration pondérale globale x + y mais dans des proportions non conformes à l'invention.

On a réalisé chacune de ces émulsions en dissolvant les filtres dans la phase grasse, puis en ajoutant les (co)émulsionnants dans cette phase grasse portée aux environs de 80°C, et enfin en additionnant sous agitation rapide l'eau préalablement chauffée à cette même température.

Pour chacune des formulations ainsi préparées, on a ensuite déterminé l'indice de protection (IP) qui leur était attaché. La mesure de l'indice de protection a été effectuée selon la méthode suivante (*in vivo*): on a appliqué ces formulations, à raison de 2 mg de produit / cm2 de peau, sur le dos de 5 modèles humains, puis on a soumis simultanément les zones protégées et les zones non protégées de peau à l'action d'un simulateur solaire commercialisé sous le nom de "Xénon Müller WG 305-1 mm" ; l'indice de protection (IP) a été alors calculé mathématiquement par le rapport du temps d'irradiation qui a été nécessaire pour atteindre le seuil érythématogène avec le filtre UV (zone protégée) au temps qui a été nécessaire pour atteindre le seuil érythématogène sans filtre UV (zone non protégée).

Les compositions des différentes formulations étudiées et les résultats en indice de protection moyen (moyenne sur les 5 modèles) obtenus sont rassemblés dans le tableau I donné ci-dessous :

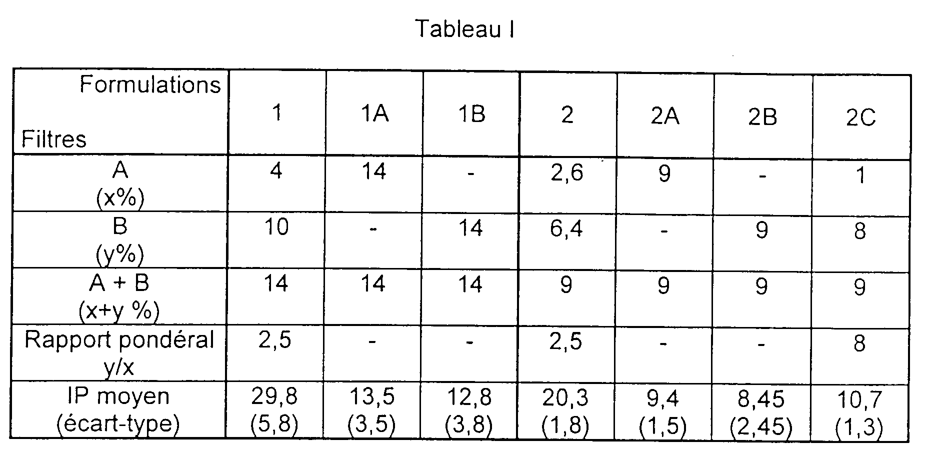

Ces résultats démontrent clairement l'effet de synergie spectaculaire obtenu avec les compositions 1 et 2 conformes à l'invention.

### EXEMPLE 2

On a préparé quatre formulations antisolaires (I, II, III, IV) se présentant sous la forme d'une émulsion de type huile-dans-eau et contenant (les quantités sont exprimées en % poids par rapport au poids total de la composition) :

| | |
|---|---|
| a) un premier filtre A qui était l'acide benzène 1,4-di(3-méthylidène-10-camphosulfonique), à raison de | x % |
| b) un deuxième filtre B qui était l'α-cyano-β,β-diphénylacrylate de 2-éthylhéxyle ("UVINUL N 539"), à raison de | y % |
| c) un troisième filtre C qui était le 4-tert.butyl-4'méthoxy-dibenzoylméthane (〈〈 PARSOL 1789 〉〉), à raison de | z % |
| d) Mélange de monostéarate de glycérol et stéarate de polyéthylène glycol à 100 moles d'oxyde d'éthylène, vendu sous la dénomination "Arlacel 165" (émulsionnant) | 1,5 % |
| e) Mélange de benzoate d'alcools en C12/C15 (〈〈 FINSOLV TN 〉〉) | 4 % |
| f) Hydratants | 15 % |
| g) Acide polyacrylique réticulé | 0,2 % |
| h) Héxadécylphosphate de potassium | 1 % |
| i) Alcool cétylique | 1,5 % |
| j) Acide stéarique | 1,5 % |
| k) Triéthanolamine | 2,7 % |
| l) Conservateurs, anti-oxydants, sequestrants | qs |
| m) Eau | qsp 100 % |

On a ensuite mesuré, selon le protocole donné à l'exemple 1, l'indice de protection (IP) attaché à chacune de ces formulations.

Les compositions des différentes formulations étudiées et les résultats en indice de protection moyen (moyenne sur les 5 modèles) obtenus sont rassemblés dans le tableau II donné ci-dessous :

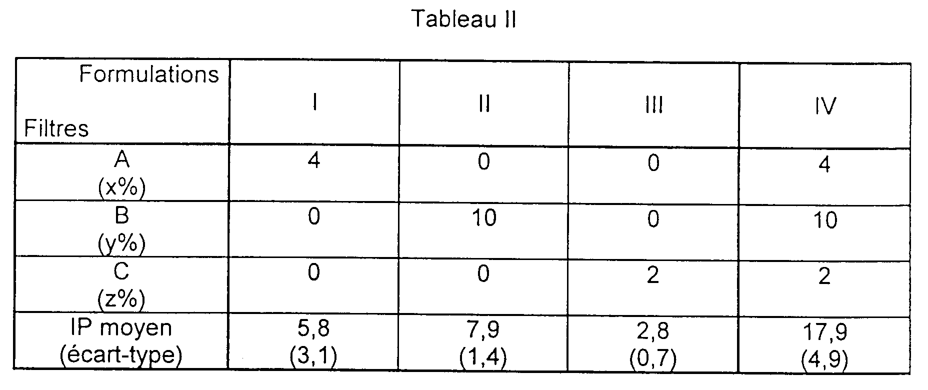

On constate donc que l'indice de protection attaché à la composition IV (égal à 17,9) n'est significativement pas, compte-tenu des écarts-types, supérieur à la simple somme arithmétique (égale à 16,5) des indices de protection attachés aux compositions A, B et C ne contenant qu'un seul filtre.

Cet exemple montre donc que l'effet de synergie attaché à l'association binaire filtrante conforme à l'invention est perdu en présence d'un troisième filtre de type PARSOL 1789.

### EXEMPLE 3

On donne ci-dessous un exemple concret de composition conforme à l'invention se présentant sous la forme d'un gel alcoolique

| | |
|---|---|
| - acide benzène 1,4-di(3-méthylidène-10-camphosulfonique) | 1,5 g |
| - α-cyano-β, β-diphénylacrylate de 2-éthylhéxyle ("UVINUL N 539") | 5 g |
| - Alcool éthylique à 96 % | 50 g |
| - Hydroxypropyl éther de cellulose (PM=3 00 000) | 1 g |
| - Adipate de diisopropyle | 4 g |
| - Glycérine | 4 g |
| - Conservateurs qs | |
| - Parfums qs | |
| - "Silicon DC 245 fluid" de chez DOW CORNING | qsp 100 g |

## Revendications

1. Compositions cosmétiques à usage topique, en particulier pour la photoprotection de la peau et/ou des cheveux, caractérisées par le fait qu'elles comprennent, dans un support cosmétiquement acceptable, (i) de l'acide benzène 1,4-di(3-méthylidène-10-camphosulfonique), éventuellement sous forme partiellement ou totalement neutralisée, à titre de premier filtre, et (ii) de l'α-cyano-β, β-diphénylacrylate de 2-éthylhéxyle à titre de second filtre, lesdits premier et second filtres étant présents dans lesdites compositions dans une proportion produisant une activité synergique.

2. Compositions selon la revendication 1, caractérisées par le fait que le rapport pondéral [(second filtre) / (premier filtre)] est compris entre 0,25 et 8.

3. Compositions selon la revendication 2, caractérisées par le fait que ledit rapport est compris entre 0,5 et 7.

4. Compositions selon la revendications 3, caractérisées par le fait que ledit rapport est compris entre 1 et 5.

5. Compositions selon l'une quelconque des revendications précédentes, caractérisées par le fait que ledit support cosmétiquement acceptable se présente sous la forme d'une émulsion de type huile-dans-eau.

6. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

7. Compositions selon la revendication 6, caractérisées par le fait que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β -diphénylacrylate, les dérivés de l'acide p-aminobenzoïque, les polymères filtres et silicones filtres

8. Compositions selon l'une quelconque des revendications précédentes, caractérisées par le fait qu'elles comprennent en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

9. Compositions selon la revendication 8, caractérisées par le fait que lesdits pigments ou nanopigments sont choisis parmi les oxydes de titane, de zinc, de fer, de zirconium, de cérium et leurs mélanges, enrobés ou non.

10. Compositions selon l'une quelconque des revendications précédentes, caractérisées par le fait qu'elles comprennent en outre au moins un agent de bronzage et/ou de brunissage artificiel de la peau.

11. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

12. Compositions selon l'une quelconque des revendications précédentes, caractérisées par le fait qu'il s'agit de compositions protectices de l'épiderme humain ou de compositions antisolaires et qu'elles se présentent sous forme de dispersions vésiculaires non ioniques, émulsions, en particulier émulsions de type huile-dans-eau, crèmes, laits, gels, gels crèmes, suspensions, dispersions, poudres, bâtonnets solides, mousses ou spray.

13. Compositions selon l'une quelconque des revendications 1 à 11, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

14. Compositions selon l'une quelconque des revendications 1 à 11, caractérisées par le fait qu'il s'agit de compositions destinées à la protection des cheveux contre les rayons ultraviolets et qu'elles se présentent sous forme de shampooings, de lotions, de gels, d'émulsions, de dispersions vésiculaires non ioniques ou de laques pour cheveux.

15. Compositions selon l'une quelconque des revendications précédentes, caractérisées par le fait qu'elles présentent un indice de protection sur peau d'au moins 2.

16. Compositions selon l'une quelconque des revendications précédentes, caractérisées par le fait que ledit premier filtre répond à la formule (I) suivante : 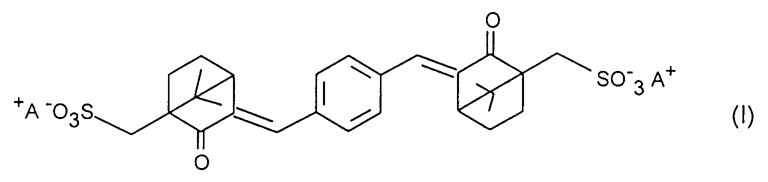 dans laquelle A désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺.

17. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

18. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 16.

## Claims

1. Cosmetic compositions for topical use, in particular for the photoprotection of the skin and/or hair, characterized in that they comprise, in a cosmetically acceptable vehicle, (i) benzene-1,4-di(3-methylidene-10-camphorsulphonic) acid, optionally in the partially or entirely neutralized form, as first screening agent, and (ii) 2-ethylhexyl α-cyano-β,β-diphenylacrylate as second screening agent, the said first and second screening agents being present in the said compositions in a proportion which produces a synergic activity.

2. Compositions according to Claim 1, characterized in that the [(second screening agent)/(first screening agent)] ratio by weight is between 0.25 and 8.

3. Compositions according to Claim 2, characterized in that the said ratio is between 0.5 and 7.

4. Compositions according to Claim 3, characterized in that the said ratio is between 1 and 5.

5. Compositions according to any one of the preceding claims, characterized in that the said cosmetically acceptable vehicle is provided in the form of an emulsion of oil-in-water type.

6. Compositions according to any one of the preceding claims, characterized in that they additionally comprise one or a number of additional, hydrophilic or lipophilic, organic screening agents which are active in the UV-A and/or UV-B.

7. Compositions according to Claim 6, characterized in that the said additional organic screening agents are chosen from cinnamic derivatives, salicylic derivatives, camphor derivatives, triazine derivatives, benzophenone derivatives, dibenzoylmethane derivatives, β, β-diphenylacrylate derivatives, p-aminobenzoic acid derivatives or polymer screening agents and silicone screening agents.

8. Compositions according to any one of the preceding claims, characterized in that they additionally comprise, as additional photoprotective agents, coated or non-coated metal oxide pigments or nanopigments capable of physically blocking, by scattering and/or reflection, the UV radiation.

9. Compositions according to Claim 8, characterized in that the said pigments or nanopigments are chosen from coated or non-coated titanium oxide, zinc oxide, iron oxide, zirconium oxide, cerium oxide and their mixtures.

10. Compositions according to any one of the preceding claims, characterized in that they additionally comprise at least one agent for the artificial bronzing and/or tanning of the skin.

11. Compositions according to any one of the preceding claims, characterized in that they additionally comprise at least one adjuvant chosen from fatty substances, organic solvents, ionic or non-ionic thickeners, softeners, antioxidants, opacifying agents, stabilizers, emollients, silicones, α-hydroxy acids, anti-foaming agents, moisturizing agents, vitamins, fragrances, preservatives, surface-active agents, fillers, sequestering agents, polymers, propellants, basifying or acidifying agents or dyes.

12. Compositions according to any one of the preceding claims, characterized in that it concerns protective compositions for the human skin or anti-sun compositions and in that they are provided in the form of non-ionic vesicular dispersions or emulsions, in particular emulsions of oil-in-water type, creams, milks, gels, cream gels, suspensions, dispersions, powders, solid sticks, foams or sprays.

13. Compositions according to any one of Claims 1 to 11, characterized in that it concerns make-up compositions for the eyelashes, eyebrows or skin and in that they are provided in the form, anhydrous or aqueous, solid or pasty, of emulsions, suspensions or dispersions.

14. Compositions according to any one of Claims 1 to 11, characterized in that it concerns compositions intended for the protection of the hair against ultraviolet radiation and in that they are provided in the form of shampoos, lotions, gels, emulsions, non-ionic vesicular dispersions or hair lacquers.

15. Compositions according to any one of the preceding claims, characterized in that they have a protection factor on the skin of at least 2.

16. Compositions according to any one of the preceding claims, characterized in that the said first screening agent corresponds to the following formula (I): 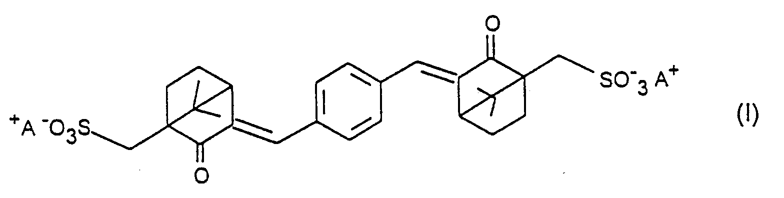 in which A denotes a hydrogen atom, an alkali metal or alternatively a radical NH(R)₃⁺ in which the R radicals, which can be identical or different, denote a hydrogen atom or a C₁-C₄ hydroxyalkyl or alkyl radical or alternatively a group Mⁿ⁺/n, Mⁿ⁺ denoting a polyvalent metal cation in which n is equal to 2 or 3 or 4, Mⁿ⁺ preferably denoting a metal cation chosen from Ca²⁺, Zn²⁺, Mg²⁺, Ba²⁺, Al³⁺ and Zr⁴⁺.

17. Use of the compositions defined in any one of the preceding claims as, or for the manufacture of, cosmetic compositions for the protection of the skin and/or hair against ultraviolet radiation and in particular solar radiation.

18. Cosmetic treatment process for protecting the skin and/or the hair against ultraviolet radiation, in particular solar radiation, characterized in that it consists in applying an effective amount of a composition as defined in any one of Claims 1 to 16 to the latter.

## Patentansprüche

1. Kosmetische Zusammensetzungen zur topischen Anwendung, insbesondere zum Lichtschutz der Haut und/oder der Haare, dadurch gekennzeichnet, daß sie in einem kosmetisch akzeptablen Träger enthalten:
(i) Benzol-1,4-di(3-methyliden-10-camphersulfonsäure), gegebenenfalls in teilweise oder vollständig neutralisierter Form, als erstes Filter und
(ii) 2-Ethylhexyl-α-cyano-β,β-diphenylacrylat als zweites Filter,
wobei das erste und das zweite Filter in den Zusammensetzungen in einem Mengenanteil vorliegen, bei dem sich eine synergistische Wirksamkeit ergibt.

2. Zusammensetzungen nach Anspruch 1, dadurch gekennzeichnet, daß das Gewichtsverhältnis (erstes Filter)/(zweites Filter) im Bereich von 0,25 bis 8 liegt.

3. Zusammensetzungen nach Anspruch 2, dadurch gekennzeichnet, daß das Gewichtsverhältnis im Bereich von 0,5 bis 7 liegt.

4. Zusammensetzungen nach Anspruch 3, dadurch gekennzeichnet, daß das Gewichtsverhältnis im Bereich von 1 bis 5 liegt.

5. Zusammensetzungen nach einem der vorhergehenden Ansprüche, dadurch gekennzeichnet, daß der kosmetisch geeignete Träger in Form einer Emulsion vom Öl-in-Wasser-Typ vorliegt.

6. Zusammensetzungen nach einem der vorhergehenden Ansprüche, dadurch gekennzeichnet, daß sie ferner ein oder mehrere zusätzliche hydrophile oder lipophile organische Filter enthalten, die im UV-A-Bereich und/oder im UV-B-Bereich wirksam sind.

7. Zusammensetzungen nach Anspruch 6, dadurch gekennzeichnet, daß die zusätzlichen organischen Filter ausgewählt sind unter Zimtsäurederivaten, Salicylsäurederivaten, Campherderivaten, Triazinderivaten, Benzophenonderivaten, Dibenzoylmethanderivaten, β,β-Diphenylacrylat-Derivaten, Derivaten der p-Aminobenzoesäure, polymeren Filtern und Siliconfiltern.

8. Zusammensetzungen nach einem der vorhergehenden Ansprüche, dadurch gekennzeichnet, daß sie ferner als zusätzliche Lichtschutzmittel Pigmente oder Nanopigmente von Metalloxiden, die gegebenenfalls ummantelt sind, enthalten, die in der Lage sind, UV-Strahlung durch Streuung und/oder Reflexion physikalisch zu blockieren.

9. Zusammensetzungen nach Anspruch 8, dadurch gekennzeichnet, daß die Pigmente oder Nanopigmente unter Oxiden von Titan, Zink, Eisen, Zirconium und Cer sowie deren Gemischen ausgewählt und gegebenenfalls ummantelt sind.

10. Zusammensetzungen nach einem der vorhergehenden Ansprüche, dadurch gekennzeichnet, daß sie ferner mindestens ein Mittel zur künstlichen Bräunung und/oder Braunfärbung der Haut enthalten.

11. Zusammensetzungen nach einem der vorhergehenden Ansprüche, dadurch gekennzeichnet, daß sie ferner mindestens einen Hilfsstoff enthalten, der ausgewählt ist unter Fettstoffen, organischen Lösungsmitteln, ionischen oder nichtionischen Verdickungsmitteln, Mitteln für die Geschmeidigkeit, Antioxidationsmitteln, Trübungsmitteln, Stabilisierungsmitteln, Emollientien, Siliconen, α-Hydroxysäuren, Antischaummitteln, Hydratisierungsmitteln, Vitaminen, Parfums, Konservierungsmitteln, grenzflächenaktiven Mitteln, Füllstoffen, Maskierungsmitteln, Polymeren, Treibmitteln, Mitteln zum Alkalischmachen, Ansäuerungsmitteln und Färbemitteln.

12. Zusammensetzungen nach einem der vorhergehenden Ansprüche, dadurch gekennzeichnet, daß es sich um Zusammensetzungen zum Schutz der menschlichen Epidermis oder Zusammensetzungen zum Sonnenschutz handelt und diese in Form von nichtionischen Vesikeldispersionen, Emulsionen, insbesondere Emulsionen vom Öl-in-Wasser-Typ, Cremen, Milchen, Gelen, Gelcremen, Suspensionen, Dispersionen, Pulvern, festen Stiften, Schäumen oder Sprays vorliegen.

13. Zusammensetzungen nach einem der Ansprüche 1 bis 11, dadurch gekennzeichnet, daß es sich um Zusammensetzungen zum Schminken der Wimpern, der Augenbrauen oder der Haut handelt und sie in fester oder pastoser, wasserfreier oder wasserhaltiger Form als Emulsionen, Suspensionen oder Dispersionen vorliegen.

14. Zusammensetzungen nach einem der Ansprüche 1 bis 11, dadurch gekennzeichnet, daß es sich um Zusammensetzungen zum Schutz der Haare gegen ultraviolette Strahlung handelt und sie in Form von Shampoos, Lotionen, Gelen, Emulsionen, nichtionischen Vesikeldispersionen oder Haarlacken. vorliegen.

15. Zusammensetzungen nach einem der vorhergehenden Ansprüche, dadurch gekennzeichnet, daß sie einen Lichtschutzfaktor auf der Haut von mindestens 2 aufweisen.

16. Zusammensetzungen nach einem der vorhergehenden Ansprüche, dadurch gekennzeichnet, daß das erste Filter die nachstehende Formel (I) aufweist, 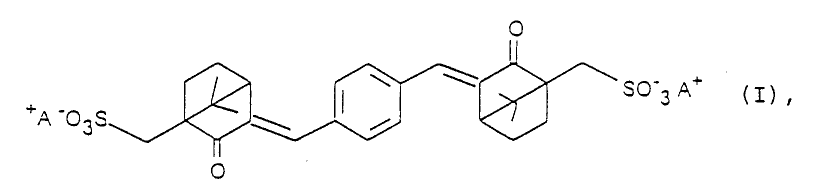 in der A ein Wasserstoffatom, ein Alkalimetall oder eine Gruppe NH(R)₃⁺ bedeutet, in der die Substituenten R, die gleich oder verschieden sind, ein Wasserstoffatom, C₁₋₄-Alkyl, C₁₋₄-Hydroxyalkyl oder eine ionische Gruppe Mⁿ⁺/n bedeuten, wobei Mⁿ⁺ ein mehrwertiges Metallkation, bei dem n gleich 2, 3 oder 4 ist, und vorzugsweise ein Metallkation darstellt, das unter Ca²⁺, Zn²⁺, Mg²⁺, Ba²⁺, Al³⁺ und Zr⁴⁺ ausgewählt ist

17. Verwendung der Zusammensetzungen nach einem der vorhergehenden Ansprüche als kosmetische Zusammensetzungen oder zur Herstellung von kosmetischen Zusammensetzungen zum Schutz der Haut und/oder der Haare gegen ultraviolette Strahlung, insbesondere gegen Sonnenstrahlung.

18. Verfahren zur kosmetischen Behandlung zum Schutz der Haut und/oder der Haare gegen Ultraviolettstrahlung, insbesondere Sonnenstrahlung, dadurch gekennzeichnet, daß es in der Anwendung einer ausreichenden Menge einer Zusammensetzung nach einem der Ansprüche 1 bis 16 auf die Haut und/oder die Haare besteht.
